# EUROPEAN PATENT SPECIFICATION

(11) **EP 3 860 378 B1**
(45) Date of publication and mention of the grant of the patent: **30.11.2022**
(21) Application number: 19787382.1
(22) Date of filing: 04.10.2019
(51) Int. Cl.: A24F 40/30

(54) **CARTOMISER**
CARTOMISER
CARTOMISEUR

(30) Priority: 05.10.2018 GB 201816267
(43) Date of publication of application: 11.08.2021
(73) Proprietor: Nicoventures Trading Limited, London WC2R 3LA (GB)
(72) Inventor: HOWARD, Matthew, London WC2R 3LA (GB); BISHOP, David, London WC2R 3LA (GB); RUSHFORTH, David, London WC2R 3LA (GB); NANDRA, Chaz, London WC2R 3LA (GB)
(74) Representative: D Young & Co LLP
(86) International application number: PCT/GB2019/052804
(87) International publication number: WO 2020/070513

(56) References cited:
- WO-A1-2015/189556
- WO-A1-2018/146453

## Description

### FIELD

The present disclosure relates to a cartomiser for a vapour provision system such as a cartridge for a nicotine delivery system, electronic cigarettes or the like, a vapour provision system comprising the cartomiser, and use of a first and second vapour precursor material respectively having a first and a second flavour characteristic, to provide different flavour characteristics to a user of a vapour provision system. In particular, the present disclosure relates to the use of the first and second vapour precursor material to provide an end-of-life tobacco flavour to a user. WO2018/146453A1 discloses a known cartomiser for a vapour provision system configured to selectively generate vapours with different flavour characteristics.

### BACKGROUND

Electronic vapour provision systems such as electronic cigarettes (e-cigarettes) generally contain a cartomiser with a reservoir of a liquid containing a formulation, typically including nicotine and often flavourants or flavouring agents, and/or a solid material such as a tobacco-based product, from which vapour is generated for inhalation by a user, for example through heat vaporisation.

Thus, a vapour provision system will typically comprise a cartomiser with a vaporiser, e.g. a heating element, arranged to vaporise a portion of precursor material to generate vapour in a vapour generation chamber. As a user inhales on the system or device and electrical power is supplied to the vaporiser, air is drawn into the device through inlet holes and into the vapour generation chamber where the air mixes with the vaporised precursor material. There is typically a flow path connecting the vapour generation chamber with an opening in the mouthpiece of the device so that incoming air drawn through the vapour generation chamber continues along the flow path to the mouthpiece opening, carrying some of the vapour with it, and out through the mouthpiece opening for inhalation by the user.

Vapour provision systems may comprise a modular assembly including both reusable and replaceable cartridges or cartomisers. Typically a cartridge will comprise the consumable vapour precursor material and/or the vaporiser, while a reusable device part will comprise longer-life items, such as a rechargeable battery, device control circuitry, activation sensors and user interface features. The reusable part may also be referred to as a control unit or battery section, and replaceable cartridges that include both a vaporiser and precursor material may also be referred to as cartomisers.

Cartridges or cartomisers are electrically and mechanically coupled to a control unit for use, for example, using a screw thread or bayonet fixing with appropriately engaging electrical contacts. When the vapour precursor material in a cartridge is exhausted, or the user wishes to switch to a different cartridge having a different vapour precursor material, a cartridge may be removed from the control unit and a replacement cartridge attached in its place. Alternatively, the removed cartridge may be refilled before reattachment to the control unit.

A vapour provision system may be configured to issue user notifications, for example, a vapour provision system may comprise a controller configured to monitor an operating state for the system and to determine when a particular operating condition arises and provide a user notification in response thereto. For example, a vapour provision system may be configured to provide a user with a warning when a remaining amount of power or charge in a battery or a remaining amount of vapour precursor material in a cartridge falls below a threshold level. These kinds of user notifications are often provided using an indicator light, such as a light emitting diode, mounted on the device.

### SUMMARY

According to a first aspect of the invention, there is provided a cartomiser for a vapour provision system configured to selectively generate vapours with different flavour characteristics, wherein the cartomiser comprises: (i) a reservoir comprising a first vapour precursor material having a first flavour characteristic, and a second vapour precursor material having a second flavour characteristic; and (ii) at least one vaporiser for generating vapour from the first vapour precursor material and the second vapour precursor material; wherein the first vapour precursor material and the second vapour precursor material are configured to form an inhomogeneous mixture within the reservoir; wherein the first vapour precursor material is configured to provide vapour with the first flavour characteristic; and wherein the second vapour precursor material is configured to provide vapour with the second flavour characteristic, wherein the second flavour characteristic is different from the first flavour characteristic.

According to a further aspect of the invention, there is provided a vapour provision system comprising the cartomiser defined herein.

According to another further aspect of the invention, there is provided a use of a first vapour precursor material and a second vapour precursor material in a vapour provision system, to provide an end-of-life flavour; wherein the first and second vapour precursor materials form an inhomogeneous mixture; wherein the first vapour precursor material is configured to provide vapour with a first flavour characteristic; wherein the second vapour precursor material is configured to provide vapour with a second flavour characteristic that is different from the first flavour characteristic; wherein the second vapour precursor material is configured to provide vapour after at least a substantial portion of the first vapour precursor material has been vaporized, and wherein the second flavour characteristic of the second vapour precursor material comprises a tobacco flavour.

According to another further aspect of the invention, there is provided a method of operating a vapour provision system configured to selectively generate vapours with different flavour characteristics for inhalation by a user, wherein the method comprises generating a vapour from a first vapour precursor material having a first flavour characteristic, and generating a vapour from a second vapour precursor material having a second flavour characteristic that is different from the first flavour characteristic, wherein the first vapour precursor material and the second vapour precursor material are inhomogeneous, and wherein the second vapour precursor material is configured to provide vapour after at least a portion of the first vapour precursor material has been vaporized.

According to another further aspect of the invention, there is provided a method of operating a vapour provision system configured to selectively generate vapours with different flavour characteristics for inhalation by a user, wherein the method comprises generating a vapour from a first vapour precursor material having a first flavour characteristic, and generating a vapour from a second vapour precursor material having a second flavour characteristic that is different from the first flavour characteristic, wherein the first vapour precursor material and the second vapour precursor material are inhomogeneous, and wherein the second vapour precursor material is configured to provide vapour with the second flavour characteristic in response to a user action on the vapour provision system.

These and further aspects of the invention are set out in the appended independent and dependent claims. It will be appreciated that features of the dependent claims may be combined with each other and with features of the independent claims in combinations other than those explicitly set out in the claims. Furthermore, the approaches described herein are not restricted to specific embodiments such as those set out below, but include and contemplate any appropriate combinations of features presented herein. For example, a cartomiser may be provided in accordance with approaches described herein which includes any one or more of the various features described below as appropriate.

### BRIEF DESCRIPTION OF THE DRAWINGS

Embodiments will now be described, by way of example only, with reference to the accompanying drawings, in which:

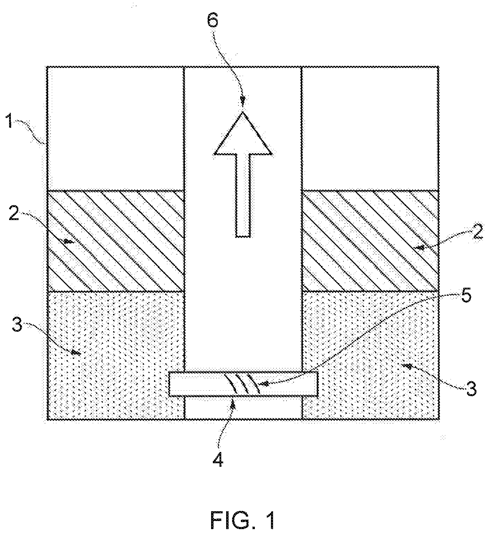

Figure 1 is a highly schematic cross-section of a cartomiser in accordance with certain embodiments of the disclosure. As is discussed in more detail below, Figure 1 shows the first vapour precursor material 3 as being in contact with the vaporiser (4, 5) and the second vapour precursor material 2 atop the first vapour precursor material.

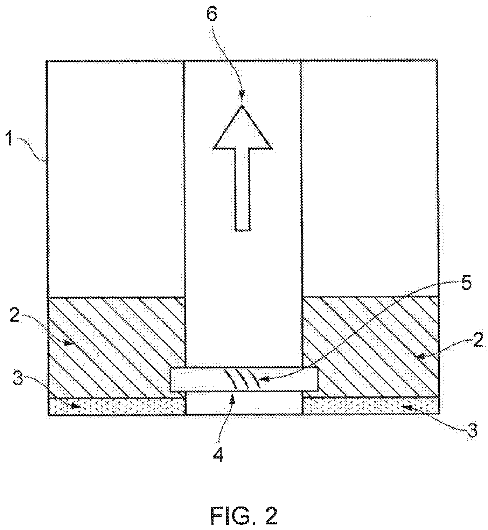

Figure 2 is a highly schematic cross-section of a cartomiser in accordance with certain embodiments of the disclosure. In Figure 2, the first vapour precursor material 3 is depleted and the second vapour precursor material is in contact with the vaporiser (4, 5).

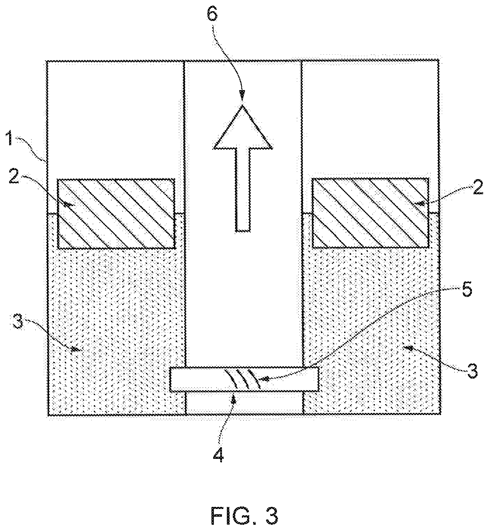

Figure 3 is a highly schematic cross-section of a cartomiser in accordance with certain embodiments of the disclosure. In Figure 3, the first vapour precursor material 3 is a different state of matter to the second vapour precursor material 2. In the embodiment shown in Figure 3, the first vapour precursor material is a liquid and the second vapour precursor material is a solid.

It will be appreciated by the person skilled in the art that none of Figures 1 to 3 are drawn to scale.

### DETAILED DESCRIPTION

Aspects and features of certain examples and embodiments are discussed and described herein. Some aspects and features of certain examples and embodiments may be implemented conventionally and these are not discussed or described in detail in the interests of brevity. It will thus be appreciated that aspects and features of apparatus and methods discussed herein which are not described in detail may be implemented in accordance with any conventional techniques for implementing such aspects and features.

As discussed herein, the present invention provides a cartomiser and a vapour provision system comprising the cartomiser which is able to provide different flavour characteristics to a user. Such flavour characteristics can include an end-of-life flavour, whether in the form of a tobacco flavour or non-tobacco flavour. These flavour characteristics can be provided without user involvement and provide a straightforward yet advantageous approach to improving user experience.

In arriving at the present invention, the inventors recognised that the known approaches for providing user notifications in vapour provision systems can have some drawbacks. For example, the provision of an indicator light can increase manufacturing complexity and associated costs, and requires visual attention from users. In addition, known approaches for providing user notification of a cartridge needing replacement can often be accompanied by an unpleasant or undesirable flavour due to the vapour precursor material reaching a critical level in the reservoir. Accordingly there is a desire for alternative schemes for providing user notifications in vapour provision systems, and specifically a desire for vapour provision systems to be able to provide an "end-of-life" notification to a user without relying on visual attention from users.

The inventors further recognised that vapour provision systems containing a solid tobacco product and a liquid have drawbacks in terms of flavour loss when the cartridge or cartomiser needs replacing. In particular, when the solid tobacco product reaches exhaustion, the user experiences a loss of flavour therefrom. Accordingly there is a desire to provide an end-of-life tobacco flavour in a vapour provision system containing both a liquid formulation and a solid tobacco product or material.

Finally, there is a desire for alternative schemes for providing multiple flavours to a user. Known approaches for delivering multiple flavours include having multiple flavour-containing reservoirs in a cartridge, optionally with user control of the end flavour which is inhaled. Such approaches are, however, complex in terms of manufacture and require user involvement in order to deliver a particular flavour profile.

As well as providing an end-of-life flavour without requiring user involvement, the present invention is able to compensate for the loss of flavour in a hybrid device, and also provides a cartomiser and a vapour provision system comprising the cartomiser which can be easily implemented by a user to provide a boost or burst of flavour. Specifically the present inventors found that these advantages can be achieved by incorporating a first vapour precursor material having a first flavour characteristic and a second vapour precursor material having a second flavour characteristic into a reservoir of a cartomiser, where the vapour precursor materials are configured such that they form an inhomogeneous mixture in the reservoir, and where the second flavour characteristic is different from the first flavour characteristic.

For ease of reference, these and further aspects of the present invention are now discussed under appropriate section headings. However, the teachings under each section are not necessarily limited to the section in which they are found.

### Cartomiser

The present disclosure relates to cartomisers. As is known in the art, cartomisers are also referred to as cartridges. Throughout the following description the term "cartridge" may therefore be used interchangeably with "cartomiser".

Figures 1, 2 and 3 are schematic cross-sectional and perspective views of an example cartomiser according to some embodiments of the disclosure. The cartomiser includes a housing, which in this example is formed of a plastics material. The housing supports other components of the cartomiser and also provides a mechanical interface (not shown) for connecting the cartomiser with a control unit of a vapour provision system as required. The manner by which the cartomiser connects to the control unit is not significant for the invention described herein. It may, for example, comprise a screw thread fitting or any other attachment or connection means known to the person skilled in the art. The shape of the cartomiser housing is not limited and may be any shape known in the art.

The cartomiser housing includes the reservoir that contains the first vapour precursor material 3 having a first flavour characteristic and the second vapour precursor material 2 having the second flavour characteristic. The reservoir in the example shown in Figures 1, 2 and 3 comprises the majority of the interior volume of the cartomiser. Although not significant for the present invention, the reservoir may generally conform to the interior of the housing. In some examples, at least an outer wall of the reservoir may be integrally moulded with the housing. In other examples, the reservoir may be a component which is formed separately from, but supported in position by, the housing. In examples, the reservoir may have a tapered circular cross-section but have a flat face running longitudinally along one side to create a space between an outer wall of the reservoir and an inner wall of the housing to define an air path through the cartomiser through which vapour generated in the cartomiser is drawn during use towards an opening in the end of the cartomiser. In other examples, the reservoir may have an annular shape, with the outer annular surface defined by the housing, and the inner annular surface defining an air path. It will be appreciated that there are many configurations which allow for the provision of a liquid reservoir alongside an air path within the cartomiser. The reservoir may be formed in accordance with conventional techniques, for example comprising a moulded plastics material.

Figures 1, 2 and 3 show a wick 4 and a heater 5 as the vaporiser. Other known vaporisers may, however, be used. The wick and heater are arranged in a space within the cartomiser that defines a vaporisation chamber 6 for the cartomiser 1. In this example the wick extends transversely across the vaporisation chamber 6 with its ends extending into the reservoir of liquid, e.g. vapour precursor material, through openings in the inner wall of the reservoir. The openings in the inner wall of the reservoir are sized to broadly match the dimensions of the wick to provide a reasonable seal against leakage from the liquid reservoir into the cartridge air path without unduly compressing the wick, which may be detrimental to its fluid transfer performance. Vapour precursor material, e.g. liquid, may infiltrate the wick 4 through surface tension or capillary action.

In other examples the reservoir may comprise a porous ceramic disc (not shown) such that the liquid, e.g. vapour precursor materials, within the reservoir may seep through the ceramic disc. Adjacent the ceramic disc is then the wick of the vaporiser into which the liquid may infiltrate.

The heater 5 in Figures 1, 2 and 3 comprises an electrically resistive wire coiled around the wick 4 so that electrical power may be supplied to the heater 5 to vaporise an amount of vapour precursor material drawn to the vicinity of the heater 5 by the wick 4. The heater 5 may comprise a nickel chrome alloy (Cr20Ni80) wire and the wick 4 may comprise a glass fibre bundle, but it will be appreciated that the specific vaporiser configuration is not significant to the invention herein.

The rate at which vapour precursor material is vaporised by the vaporiser will depend on the amount of power supplied to the heater 5. Accordingly, electrical power can be applied to the heater to selectively generate vapour from the first and second vapour precursor material 2, 3 in the cartomiser 1, and furthermore, the rate of vapour generation can be controlled by adjusting the power supplied to the heater 5, for example through pulse width or frequency modulation techniques.

In Figure 1, Figure 2 and Figure 3, the cartomiser includes a single vaporiser 4, 5 for generating vapour from both the first vapour precursor material 3 and the second vapour precursor material 2. In some embodiments the cartomiser includes more than one vaporiser, for example, a vaporiser for each vapour precursor material in the reservoir. In other embodiments the cartomiser includes a single vaporiser as shown in Figure 1, Figure 2 and Figure 3.

### Vapour provision system

The present disclosure provides a vapour provision system comprising the cartomiser described herein. Vapour provision systems/devices may also be referred to herein as "aerosol provision systems/devices", "aerosol delivery devices/systems", "electronic vapour provision devices/systems", "electronic aerosol provision devices/systems" or "e-cigarettes/electronic cigarettes". These terms may be used interchangeably and are intended to include non-combustible aerosol and vapour provision systems/devices (non-combustible smoking articles) such as:
electronic cigarettes or e-cigarettes that create vapour or aerosol from vapour precursor materials by heating or other techniques such as vibration,
hybrid systems that provide vapour or aerosol via a combination of vapour precursor materials and solid substrate materials, for example hybrid systems containing liquid or gel vapour precursor materials and a solid substrate material.

As is common in the technical field, the terms "vapour" and "aerosol", and related terms such as "vaporise", "volatilise" and "aerosolise", may be used interchangeably herein.

Vapour precursor materials may also be referred herein as aerosol generating materials, vapour generating materials, aerosol precursor materials and substrate materials. The vapour precursor materials are materials that are capable of generating aerosol for example when heated, irradiated or energized in any other way. In general, the vapour precursor materials may be in the form of a solid, liquid or gel which may or may not contain nicotine and/or flavourants.

In some embodiments, the vapour provision system is a hybrid system for providing a vapour by heating, but not burning, a combination of vapour forming materials. The hybrid system comprises a cartomiser of the present invention and a substrate material. The substrate material may comprise for example solid, liquid or gel which may or may not contain nicotine. In some embodiments, the hybrid system comprises a cartomiser of the invention with the first and second vapour precursor materials and an (additional) solid substrate material. The solid substrate material may be, for example, a tobacco or non-tobacco product, which may or may not contain nicotine. In some embodiments, the hybrid system comprises the first and second vapour precursor materials which are liquid or gel, and a tobacco. The hybrid system also generally includes a control unit as described below.

In some embodiments, the vapour provision system is a non-combustible smoking article such as an electronic cigarette, also known as a vaping device. The vapour provision system comprises a cartomiser of the invention, as described herein, and generally a control unit.

The control unit comprises an outer housing, an electrical power source (e.g. a battery), control circuitry for controlling and monitoring the operation of the vapour provision system, a user input button, and a mouthpiece (which may be detachable). The battery may be rechargeable and be of a conventional type, for example of the kind typically used in electronic cigarettes and other applications requiring provision of relatively high currents over a relatively short period. Similarly, the user input button (or other vapour generation function) and control circuity may be conventional. The outer housing may be formed, for example, from a plastics or metallic material. Other suitable materials are known in the art.

As will be appreciated, the vapour provision system will in general comprise various other elements associated with its operating functionality. For example, a port for charging the battery, such as a USB port or the like, and these other elements may be conventional.

In some embodiments the control circuitry is configured to control the supply of electrical power from the battery to the vaporiser(s) in the cartomiser. For example, the vapour provision system may include the cartomiser with at least one reservoir and vaporiser as described herein, where the at least one vaporiser includes a heater supplied with power from the battery. Electrical power may be supplied to the respective heaters via contacts established across the interface between the cartomiser and the control unit, for example, through sprung/pogo pin connectors, or any other configuration of electrical contacts which engage when the cartomiser is connected to the control unit.

As noted above, the rate at which vapour precursor material is vaporised will depend on the amount of power supplied to the heater. Accordingly, the rate of vapour generation can be controlled by adjusting the power supplied to the heater, for example through pulse width or frequency modulation techniques. Regardless of how the electrical power, including the relative amount of power, to be delivered to the heaters in the cartomisers is configured, when the electronic cigarette is in its normal operating mode, a user may press the button to activate the heater in accordance with the configured relative power settings. Although a user button is described, it will be appreciated that the activation of vapour generation may be based on other techniques. For example, instead of using a button to activate the supply of power to the heaters, an inhalation sensor, for example, based around a pressure sensor/microphone arranged to detect a drop in pressure when a user inhales on the device, may be used.

When the vapour generation function of the vapour provision system is activated, a user sucks/inhales on the mouthpiece to draw air through the device. Air is drawn from the environment into the device and at least a portion of this air enters the vaporisation chamber of the cartomiser. Accordingly, the incoming air flows past the heater in the vaporisation chamber while the heater is receiving electrical power from the battery in the control unit so as to generate a vapour from the relevant vapour precursor material in the vaporisation chamber. The vaporised material is then incorporated/entrained into the airflow and drawn through and out of the relevant cartomiser for inhalation by a user. Figures 1, 2 and 3 include an arrow to indicate the general direction of airflow through and out of the cartomiser for inhalation by a user.

When in a hybrid device, the vaporised material will typically contact and/or pass through the substrate material, e.g. a tobacco material, downstream of the vaporiser before inhalation of the vapour by a user. The substrate material may be located in the cartomiser (e.g. in the vaporisation chamber thereof) or in an air flow path from the outlet of the cartomiser to the mouthpiece or other outlet of the device. The latter may involve the substrate material being in a separate chamber or container which forms part of the airflow path. The person skilled in the art will be aware of suitable configurations for a hybrid device.

The vapour may be produced or released in various ways depending on the nature of the device, system or product. These include heating to cause evaporation, heating to release compounds, and vibration of a liquid or gel to create droplets.

During normal use, the control circuitry may be configured to monitor various operational aspects of the vapour provision system. For example, the control circuitry may be configured to monitor a level of power remaining in the rechargeable battery, and this may be performed in accordance with conventional techniques.

Additionally the control circuitry may be configured to estimate a remaining amount of vapour precursor material in the cartomiser, for example based on an accumulated time of usage since a new cartomiser was installed, or based on sensing the levels in the cartomiser. This may be performed in accordance with any conventional technique(s). The control circuitry may also be configured to estimate a remaining time for the substrate material in a hybrid device, for example, based on an accumulated time of usage since a new substrate material and/or cartomiser was installed, or based on sensing the number of puffs on the device. This may be performed in accordance with any conventional technique(s).

If it is determined through monitoring the operational aspects of the vapour provision system that a certain operating condition has arisen, for example, a cartomiser and/or substrate material is approaching depletion, or a battery level is falling below a predetermined threshold (which may be predefined or user set), the vapour provision system may be configured to provide a user notification according to any conventional technique(s). Although described with reference to the control circuitry, other user notifications are known in the art and may be implemented in the vapour provision system of the invention. In addition, it will be appreciated that there are many other situations in which a user notification might be desired, the invention is not limited to providing notification of low levels of liquid or substrate material or remaining battery power.

In accordance with the present invention, however, user notification via the control circuitry or otherwise is not necessary if an "end-of-life" operating condition has arisen. By the term "end-of-life" is meant that the cartomiser and/or substrate material is approaching depletion such that the vapour provision system is in the end of its useful life from the point of view of the user or consumer. In other words, the cartomiser and/or substrate material are approaching the point in time when they need to be replaced.

User notification of an "end-of-life" operating condition is provided by the first and second vapour precursor materials respectively having a first and second flavour characteristic, where the second flavour characteristic is different from the first flavour characteristic, and where the first and second vapour precursor materials are configured to form an inhomogeneous mixture in the reservoir of the cartomiser. The user notification is provided by an "end-of-life" flavour arising from the vaporisation of the second vapour precursor material.

In one embodiment, the second flavour characteristic of the second vapour precursor material is a tobacco flavour. The use of a tobacco flavour for the second vapour precursor material advantageously provides an end-of-life flavour which extends the useful life of a product whilst simultaneously providing a user notification. The extension of life by a tobacco flavour is particularly advantageous for a hybrid device comprising a cartomiser of the invention and a substrate which is tobacco.

For example, the first vapour precursor material may have a flavour characteristic for normal use and when substantially all of the first vapour precursor material has been vaporized, the second vapour precursor material is vaporised. This is the situation shown in Figure 2. The second flavour characteristic of the second vapour precursor material is then sensed by the user and interpreted as a user notification. The change in flavour characteristic can be used to indicate, for example, that the cartomiser needs replacing. The relative amounts of the first and second vapour precursor materials can, for instance, also be adjusted so that the user knows how many puffs they have left before the cartomiser is completely depleted.

In one embodiment the second vapour precursor material is included in an amount which equates to about 10 to about 20 puffs of the vapour provision system. On sensing the second flavour characteristic of the second vapour precursor material, the user knows that they have about 10 about 20 puffs before the cartomiser must be replaced or refilled. Depending on the size of the reservoir, the skilled person will be able to calculate the appropriate amount of the second vapour precursor material to deliver about 10 to about 20 puffs. For example, if the reservoir is approximately 2 ml in size and delivers approximately 166 puffs in total, the second vapour precursor material will be included at an amount of approximately 0.24 ml to deliver 20 puffs to the user.

When used in a hybrid device, the first vapour precursor material may have a flavour characteristic for normal use and be included in amount which corresponds to the number of puffs associated with the life of the substrate. For example, if the substrate is a tobacco material and included in an amount of which corresponds to approximately 160 puffs in normal use, the first vapour precursor material can included in an amount also corresponding to approximately 160 puffs in normal use. This means that when substantially all of the first vapour precursor material has been vaporised, the vaporisation of the second vapour precursor material will substantially coincide with the exhaustion of the tobacco material. The second flavour characteristic can then either be a flavour which, when sensed by the user, indicates that the substrate and cartomiser need replacing and/or a tobacco flavour to avoid the loss of flavour on exhaustion of the substrate.

Alternatively the second flavour characteristic of the second vapour precursor material can be used to provide additional flavours to a user, not necessarily associated with a user notification.

Thus, in accordance with certain embodiments, a cartomiser and a vapour provision system is provided which generates end-of-life feedback for a user without relying on control circuitry or multiple cartridges/cartomisers. Not only does this obviate the need for a separate status light indicator or complex device design/manufacture, but it can provide the user with notification without the user needing to maintain visual focus on a light indicator. This can help provide the user with end-of-life notifications in situations where an illuminating light would not be desired.

The vapour provision system may also be configured to provide a user notification by changing flavour based on a determined amount of use. For example, if the cartomiser included three or more vapour precursor materials, it may be configured to generate vapour from the second precursor material having the second flavour characteristic after a given amount of vapour has been generated from the first vapour precursor material. This would be achieved by aligning the amount of first vapour precursor material with a number of puffs. The second vapour precursor material would then indicate to the user that they have inhaled an amount of e.g. nicotine from the first vapour precursor material, and only after the second vapour precursor material had been vaporised would the third vapour precursor material having a third flavour characteristic be sensed by the user. This third vapour precursor material could, for example, have the same or different flavour characteristic to the first vapour precursor material but would have a flavour characteristic which differed from the second flavour characteristic. Further vapour precursor materials with defined flavour characteristics could also be included to provide an end-of-life notification to the user.

In accordance with the invention, all of the vapour precursor materials would be configured to form an inhomogeneous mixture.

This and other aspects of the vapour precursor material will now be discussed in more detail,

### Vapour Precursor Material

The present disclosure relates to a first vapour precursor material 3 having a first flavour characteristic, and a second vapour precursor material 2 having a second flavour characteristic. It will, however, be appreciated by the person skilled in the art that the present disclosure is not limited to two such vapour precursor materials. For example, the invention can include a third vapour precursor material having a third flavour characteristic, where the third vapour precursor material is configured to form an inhomogeneous mixture with the first and second vapour precursor materials, and where the third flavour characteristic is different from at least the second flavour characteristic. The invention can also include further vapour precursor materials with defined flavour characteristics.

In this respect, all of the features discussed for the first and second vapour precursor materials are applicable to further vapour precursor materials included in the invention.

By the term "vapour precursor material" is meant any substance which when brought into the vicinity of a vaporiser, forms a vapour or aerosol. The vapour precursor material can be a material which is known in the art.

The vapour precursor materials of the invention are configured to form an inhomogeneous mixture. In this regard, any means of forming an inhomogeneous mixture of two or more substances may be used. Such means are known by the person skilled in the art.

In one embodiment the vapour precursor materials form an inhomogeneous mixture because they are immiscible. By the term "immiscible" is meant a liquid which cannot be mixed with another liquid without separating from it. Oil is, for example, immiscible with or in water.

In a further embodiment, the second vapour precursor material is less dense than the first vapour precursor material.

The cartomiser example shown in Figures 1, 2 and 3 is where a first vapour precursor material 3 and a second vapour precursor material 2 are immiscible and where the second vapour precursor material is less dense than the first vapour precursor material. Because of their immiscibility and relative densities, the second vapour precursor material 2 sits atop the first vapour precursor material 3 within the reservoir of the cartomiser. This arrangement, together with for example, the position of the vaporiser (here a wick 4 and a coil 5) and/or the amount of each vapour precursor material in the reservoir, allows the order and timing of vaporisation of each vapour precursor material to be controlled without user involvement.

For example, when in use, a vapour provision system containing a cartomiser shown in Figure 1 will vaporise the first vapour precursor material providing vapour with a first flavour characteristic. Once the level of the first vapour precursor material is depleted by an amount which brings the second vapour precursor material into contact with the wick, such as the arrangement shown in Figure 2, the second vapour precursor material will be vaporised to form a vapour with a second flavour characteristic. The skilled person will appreciate that if the vaporiser (e.g. wick and coil) is located at a higher position within the reservoir, less of the first vapour precursor material will need to vaporise before the second vapour precursor material is vaporised. The skilled person will also appreciate that if the first vapour precursor material amounts to e.g. about 90 to about 95 wt% of the total material in the reservoir and the second vapour precursor material amounts to e.g. about 5 to about 10 wt% of the total material in the reservoir, the user will only experience the flavour characteristic of the second vapour precursor material towards the end of the life of the cartomiser.

As well as the configuration of the vaporiser and vapour precursor materials, other means of controlling the order of vaporisation can be used. For example when the cartomiser includes a single vaporiser which is a wick and coil, different wicking rates of the first and second vapour precursor material can be used to ensure vaporisation of the first vapour precursor material preferentially over the second vapour precursor material.

In one embodiment the second vapour precursor material is configured to provide vapour after at least a portion of the first vapour precursor material has been vaporized. In one embodiment the second vapour precursor material is configured to provide vapour after substantially all of the first vapour precursor material has been vaporized.

By the term "substantially all" is meant at least about 90%,

In one embodiment the second vapour precursor material is configured to provide vapour after at least about 80% of the first vapour precursor material has been vaporised. In one embodiment the second vapour precursor material is configured to provide vapour after at least about 85% of the first vapour precursor material has been vaporised. In one embodiment the second vapour precursor material is configured to provide vapour after at least about 90% of the first vapour precursor material has been vaporised. In one embodiment the second vapour precursor material is configured to provide vapour after at least about 95% of the first vapour precursor material has been vaporised. In one embodiment the second vapour precursor material is configured to provide vapour after at least about 96% of the first vapour precursor material has been vaporised. In one embodiment the second vapour precursor material is configured to provide vapour after at least about 97% of the first vapour precursor material has been vaporised. In one embodiment the second vapour precursor material is configured to provide vapour after at least about 98% of the first vapour precursor material has been vaporised. In one embodiment the second vapour precursor material is configured to provide vapour after at least about 99% of the first vapour precursor material has been vaporised.

In one embodiment the second vapour precursor material amounts to about 1 to about 30 wt% of the total material in the reservoir. In one embodiment the second vapour precursor material amounts to about 2 to about 25 wt% of the total material in the reservoir. In one embodiment the second vapour precursor material amounts to about 3 to about 20 wt% of the total material in the reservoir. In one embodiment the second vapour precursor material amounts to about 4 to about 15 wt% of the total material in the reservoir. In one embodiment the second vapour precursor material amounts to about 5 to about 10 wt% of the total material in the reservoir.

As the skilled person will appreciate the amount of second vapour precursor material in the reservoir can be combined with the amount of first vapour precursor material that must be vaporised before the second vapour precursor material is vaporised. For example, in one embodiment the second vapour precursor material amounts to about 1 to about 30 wt% of the total material in the reservoir and is configured to provide vapour after at least about 85 wt% of the first vapour precursor material has been vaporised. In another embodiment the second vapour precursor material amounts to about 4 to about 15 wt% of the total material in the reservoir and is configured to provide vapour after at least about 90 wt% of the first vapour precursor material has been vaporised.

From the description herein, the skilled person will appreciate that whilst the order and timing of vapour being generated from each of the first and second vapour precursor material does not require user involvement, if a user were to invert the cartridge/cartomiser so that the first vapour precursor material sat atop the second vapour precursor material, vapour from the second vapour precursor material having the second flavour characteristic would be produced. The cartomiser of the invention does not therefore require user involvement, but advantageously allows a user to change the flavour characteristic of the vapour being produced, if so desired.

In one embodiment the second vapour precursor material is therefore configured to provide vapour with the second flavour characteristic in response to a user action on the vapour provision system comprising the cartomiser as disclosed herein.

When the vapour precursor materials are immiscible, they can comprise an aqueous solution and an oil solution. For example, the first vapour precursor material can be aqueous and the second vapour precursor material can comprise an oil. Alternatively, the first vapour precursor material can comprise an oil and the second vapour precursor material can be aqueous. Alternatively, other immiscible liquids known in the art can be used as the first and second vapour precursor materials respectively. The present disclosure is not limited to an oil/water system.

By the term "aqueous" is meant an aqueous solution or a solution in which the solvent is water. In one embodiment the aqueous solution comprises water and one or more compounds selected from propylene glycol, glycerol, 1,3-propanediol and mixtures thereof. In one embodiment the aqueous solution comprises water and at least one of propylene glycol and glycerol. In one embodiment the aqueous solution comprises water, propylene glycol and glycerol.

For example, the first vapour precursor material may comprise water and one or more compounds selected from propylene glycol, glycerol, 1,3-propanediol and mixtures thereof. In one embodiment the first vapour precursor material comprises water and at least one of propylene glycol and glycerol.

In another embodiment the aqueous solution comprises one or more compounds selected from propylene glycol, glycerol, 1,3-propanediol and mixtures thereof. In one embodiment the aqueous solution comprises at least one of propylene glycol and glycerol. For example, the first vapour precursor material may comprise one or more compounds selected from propylene glycol, glycerol, 1,3-propanediol and mixtures thereof. In one embodiment the first vapour precursor material comprises at least one of propylene glycol and glycerol.

By the term "oil" is meant a hydrophobic and nonpolar chemical substance that is a liquid at ambient temperatures, non-volatile and lipophilic. The oil may be animal or vegetable in origin. The skilled person would appreciate what oils are suitable for use in a vapour provision system such as described herein. In one embodiment the oil is a low molecular weight oil. By the term "low molecular weight" is meant a molecular weight low enough for the oil to be atomised within the vapour provision system. Again, the skilled person will be aware of materials which fall within this definition. In one embodiment the oil is triacetin.

In one embodiment the second vapour precursor material is an oil as defined above,

In another embodiment the vapour precursor materials form an inhomogeneous mixture because they are different states of matter; for instance, the first vapour precursor material can be a different state of matter to the second vapour precursor material. For example, the first vapour precursor material can be a liquid or a gel and the second vapour precursor material can be a solid, or vice versa. Alternatively, the first vapour precursor material can be a liquid and the second vapour precursor material can be a solid or a gel, or vice versa.

This is the situation shown in Figure 3: the first vapour precursor material is a liquid and the second vapour precursor material is a solid.

In one embodiment the second vapour precursor material is a solid or a gel. When the second vapour precursor material is a solid or a gel, it can have a melting temperature which is below the operating temperature of the vapour provision system in which the cartomiser is used.

In one embodiment the second vapour precursor material is a solid or a gel having a melting temperature below about 200°C. In another embodiment the second vapour precursor material is a solid or a gel having a melting temperature below about 190°C. In another embodiment the second vapour precursor material is a solid or a gel having a melting temperature below about 180°C. In another embodiment the second vapour precursor material is a solid or a gel having a melting temperature below about 170°C. In another embodiment the second vapour precursor material is a solid or a gel having a melting temperature below about 160°C. In another embodiment the second vapour precursor material is a solid or a gel having a melting temperature below about 150°C. In another embodiment the second vapour precursor material is a solid or a gel having a melting temperature below about 140°C. In another embodiment the second vapour precursor material is a solid or a gel having a melting temperature below about 130°C. In another embodiment the second vapour precursor material is a solid or a gel having a melting temperature below about 120°C. In another embodiment the second vapour precursor material is a solid or gel having a melting temperature below about 100°C.

In one embodiment the first vapour precursor material is an aqueous solution and the second vapour precursor material is a solid or a gel, preferably a solid having a melting temperature as defined above.

With a solid or gel second precursor material, the first vapour precursor material may be an aqueous solution comprising water and one or more compounds selected from propylene glycol, glycerol, 1,3-propanediol and mixtures thereof. In one embodiment the aqueous solution comprises water and at least one of propylene glycol and glycerol. In one embodiment the aqueous solution comprises water, propylene glycol and glycerol.

In one embodiment the first vapour precursor material is an aqueous gel and the second vapour precursor material is a solid, preferably a solid having a melting temperature as defined above.

Without wishing to be bound by any one theory, the inventors believe that by using a solid or a gel with a melting temperature below the operating temperature of the vapour provision system, it is possible for the second vapour precursor material to only be vaporised after substantially all of the first vapour precursor material has been vaporised. This is because it is only after substantially all of the first vapour precursor material has been vaporised that the second vapour precursor material comes into the vicinity of the vaporiser and undergoes a phase transition to a liquid so that it can be vaporised.

When the second vapour precursor material is a solid, it may be in any form suitable for incorporation into the reservoir of the cartomiser. The second vapour precursor material may, for example, be in the form of beads, granules, powder, a tablet, or the like. When in the form of a powder, the second vapour precursor material may be loaded onto a substrate and the combination of the substrate and powder are preferably less dense than the first vapour precursor material such that the vaporisation of the second vapour precursor material and optionally the substrate, takes place after at least partial vaporisation of the first vapour precursor material.

The substrate for the second vapour precursor material may also be vaporisable, and may have a melting temperature which is higher or lower than that of the second vapour precursor material. When the melting temperature of the second vapour precursor material is lower than the substrate, the second vapour precursor material should liquefy and then vaporise before the substrate and thereby deliver the second flavour characteristic to the user following vaporisation of substantially all of the first vapour precursor material. When the melting temperature of the substrate is lower than the melting temperature of the second vapour precursor material, the substrate should liquefy before the second vapour precursor material. In this instance, the substrate may be miscible with the first vapour precursor material.

The substrate for the second vapour precursor material is not limited and the skilled person would be readily able to identify a suitable material based on the desired properties and vaporisation behaviour of the substrate and second vapour precursor material. For example, when it is not desired for the substrate to vaporise, but merely act as a carrier for the second vapour precursor material, it may be a materia! that can absorb and then subsequently release the second vapour precursor material on application of heat or the like. For example, the substrate may be a plastic film with pores or micro-holes, a sponge-like film, a porous carbon material, a porous ceramic material, a silicon compound such as glass, or a fibrous element. Possible materials therefore include cellulose acetate, polyurethane, vinyl acetate, polycarbonate, carbon, ceramics, silicon compounds (e.g. silica) or mixtures thereof. In various embodiments, the material may be a ceramic material, a carbon material, or a glass.

Alternatively when it is desired for the substrate to vaporise at least partially, it may either have a higher melting temperature than the second vapour precursor material or have a melting temperature equal to or less than the melting temperature of the second vapour precursor material and be miscible with the first vapour precursor material upon liquefying. When the substrate is vaporisable it should not have a significant effect on the flavour characteristic of the second vapour precursor material. The substrate may therefore comprise a material selected from the group consisting of carboxymethyl cellulose, pullulan, hydroxypropylmethyl cellulose, hydroxyethyl cellulose, hydroxypropyl cellulose, polyvinyl pyrrolidone, polyvinyl alcohol, sodium alginate, polyethylene glycol, xanthum gum, tragacanth gum, guar gum, acacia gum, Arabic gum, natural waxes, shea butter, carbowax, polyacrylic acid, methylmethacrylate copolymer, carboxyvinyl polymer, carrageenan, amylose, high amylose starch, hydroxypropylated high amylose starch, dextrin, pectin, chitin, chitosan, levan, elsinan, collagen, gelatin, zein, gluten, soy protein isolate, whey protein isolate, casein and/or mixtures thereof.

In another embodiment, the vapour precursor materials form an inhomogeneous mixture in the reservoir because a layer or barrier is included which separates the first vapour precursor material from the second vapour precursor material. Such a layer or barrier may separate the materials physically or chemically. By "physically or chemically" is meant that the layer may act as a physical barrier between the materials, or the layer may have chemical properties which prevent the materials from forming a homogenous mixture. Regardless of whether the layer physically or chemically separates the materials, the layer should be composed of a material which does not interfere with or have a detrimental effect on the cartomiser or the vapour provision system, when the cartomiser is in use.

For example, the layer may be made of a material which is able to be vaporised by the vaporiser in the cartomiser. In one embodiment, the layer is an oil. It will be appreciated by the skilled person that when the cartomiser includes a layer to separate the vapour precursor materials and form an inhomogeneous mixture, it may be necessary to include a filter in the cartomiser to prevent vaporisation and inhalation of the oil by a user. Suitable filters are known in the art.

In various embodiments of the present disclosure, the layer or barrier separating the first and second vapour precursor materials is in the form of a matrix or shell, for example, the second vapour precursor material may be encapsulated in a matrix material or encapsulated with an outer shell. In this capsule arrangement, the second vapour precursor material may be a liquid, gel or solid.

In various embodiments of the present disclosure, the shell or matrix comprising the second vapour precursor material is configured to degrade, rupture or melt in response to an increase in temperature. The increase in temperature is caused by exhaustion of the first vapour precursor material, and the shell or matrix being in contact with or close to the heating element of the cartomiser. For example, the shell or matrix may be partially or completely formed from a material which degrades, ruptures or melts in response to the increase in temperature.

In various embodiments of the present disclosure, the shell or matrix is formed from a material which degrades, ruptures or melts at a temperature of equal to or less than about 100°C. For example a material which has a melting temperature of greater than about 20°C and equal to or less than about 100°C, such as a material which has a melting temperature of between about 40°C and about 100°C. Suitable materials are known in the art and may include various waxes, resins and high molecular weight polyethylene glycols or the like.

Without wishing to be bound by theory, the inventors believe that such a capsule arrangement involves the shell or matrix material degrading, rupturing or melting to thereby release the second vapour precursor material. The shell or matrix material may be miscible with the first vapour precursor material and optionally be vaporised with the first vapour precursor material.

The combination of shell or matrix material and second vapour precursor material should be less dense than the first vapour precursor material. With this arrangement, the capsule formed by the shell/matrix and the second vapour precursor material effectively "floats" on the surface of the first vapour precursor material. Upon degradation, rupture or melting of the shell/matrix material, the second vapour precursor material is then released and vaporised. For example, the first vapour precursor material may be a solution comprising propylene glycol, glycerol, 1,3-propanediol or mixtures thereof, and the second vapour precursor material may be an aqueous solution, an ethanol solution or a polyethylene glycol solution.

In particular, the shell/matrix material may comprise a material selected from the group consisting of hydroxypropyl cellulose, high molecular weight polyethylene glycols (e.g. a PEG with a molecular weight greater than 2000 or greater than 2500 such as PEG 3350, PEG 4000, PEG 6000 and the like), natural waxes, shea butter, carbowax, carrageenan, collagen, gelatin, whey protein isolate, casein and/or mixtures thereof. In various embodiments of the present disclosure, the shell/matrix material is selected from the group consisting of polyethylene glycol, natural waxes, shea butter, carbowax carrageenan, or mixtures thereof.

### Flavour Characteristic

The vapour precursor materials of the invention have different flavour characteristics. In one embodiment these flavour characteristics can be achieved by including or equally not-including a "flavour", "flavouring agent" or "flavourant" in the vapour precursor material. The terms "flavour", "flavouring agent" and "flavourant" are used interchangeably to refer to materials which, where local regulations permit, are added to the formulation to create a desired taste or aroma in a product for adult consumers. Reference here to "flavour", "flavouring agent" or "flavourant" include both singular and multi-component flavours.

It will, however, be appreciated that the specific nature of the flavours used in accordance with the invention is not necessarily significant. For example, in one embodiment the actual flavours of the vapour precursor materials are not significant and can include each vapour precursor material having the same flavour but at different strengths/levels. What is significant is that a characteristic of the flavour associated with a vapour changes. Thus a change in flavour characteristic may comprise, for example, a change in actual flavour, a change in strength of a flavour, or a change in whether there is any flavour.

In some embodiments, one of the vapour precursor materials may be flavoured and the other liquid may not be flavoured. For example, in one embodiment the first vapour precursor material does not include a flavour - the first vapour precursor material is a non-flavoured vapour precursor material - and the second vapour precursor material includes a flavour. In one embodiment the second vapour precursor material includes a tobacco flavour. In another embodiment the second vapour precursor material includes a non-tobacco flavour.

When a flavour, flavouring agent or flavourant is included in the vapour precursor material(s) to provide a flavour characteristic, this flavour, flavouring agent or flavourant may be selected from the group consisting of extracts, for example liquorice, hydrangea, Japanese white bark magnolia leaf, tobacco, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, pimento, ginger, anise, coriander, coffee, flavour enhancers, bitterness receptor site blockers, sensorial receptor site activators or stimulators, sugars and/or sugar substitutes (e.g. sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder.

In one embodiment the second vapour precursor material includes a flavour which indicates to a user that the cartridge is nearing the end of its life. This indicative flavour could be e.g. menthol, such that when the user inhales vapour with a menthol flavour, they know to replace the cartomiser with a fresh cartomiser.

In another embodiment the second vapour precursor material includes a flavour which extends the life of the vapour provision system as well as providing an indication to a user that the cartridge is nearing the end of it life. This flavour could be e.g. a tobacco flavour.

### Other components

Along with having a certain flavour characteristic, the vapour precursor materials may comprise other components. Such components may be conventional in the sense that they are typically included in vapour precursor materials for e-cigarettes and the like.

In one embodiment the vapour precursor materials further comprise an active agent. By the term "active agent" is meant any agent which has a biological effect on a subject when the vapour is inhaled. The one or more active agents may be selected from nicotine, botanicals, and mixtures thereof. The one or more active agents may be of synthetic or natural origin. The active could be an extract from a botanical, such as from a plant in the tobacco family, An example active is nicotine.

Thus in one embodiment the first vapour precursor material comprises an active agent; preferably the active agent is nicotine. Nicotine may be provided in any suitable amount depending on the desired dosage when inhaled by the user.

In one embodiment nicotine is present in an amount of no greater than about 6 wt% based on the total weight of the vapour precursor material. By the expression "total weight of the vapour precursor material" is meant the total weight of the vapour precursor material in which the nicotine is present, e.g. the first vapour precursor material.

In one embodiment nicotine is present in an amount of from about 0.4 to about 6 wt% based on the total weight of the vapour precursor material. In one embodiment nicotine is present in an amount of from about 0.8 to about 6 wt% based on the total weight of the vapour precursor material. In one embodiment nicotine is present in an amount of from about 1 to about 6 wt% based on the total weight of the vapour precursor material. In one embodiment nicotine is present in an amount of from about 1.8 to about 6 wt% based on the total weight of the vapour precursor material.

In another embodiment nicotine is present in an amount of no greater than about 3 wt% based on the total weight of the vapour precursor material. In one embodiment nicotine is present in an amount of from about 0.4 to about 3 wt% based on the total weight of the vapour precursor material. In one embodiment nicotine is present in an amount of from about 0.8 to about 3 wt% based on the total weight of the vapour precursor material. In one embodiment nicotine is present in an amount of from about 1 to about 3 wt% based on the total weight of the vapour precursor material. In one embodiment nicotine is present in an amount of from about 1.8 to about 3 wt% based on the total weight of the vapour precursor material.

In one embodiment nicotine is present in an amount of less than about 1.9 wt% based on the total weight of the vapour precursor material. In one embodiment nicotine is present in an amount of less than about 1.8 wt% based on the total weight of the vapour precursor material. In one embodiment nicotine is present in an amount of from about 0.4 to less than about 1.9 wt% based on the total weight of the vapour precursor material. In one embodiment nicotine is present in an amount of from about 0.4 to less than about 1.8 wt% based on the total weight of the vapour precursor material. In one embodiment nicotine is present in an amount of from about 0.5 to less than about 1.9 wt% based on the total weight of the vapour precursor material. In one embodiment nicotine is present in an amount of from about 0.5 to less than about 1.8 wt% based on the total weight of the vapour precursor material. In one embodiment nicotine is present in an amount of from about 0.8 to less than about 1.9 wt% based on the total weight of the vapour precursor material. In one embodiment nicotine is present in an amount of from about 0.8 to less than about 1.8 wt% based on the total weight of the vapour precursor material. In one embodiment nicotine is present in an amount of from about 1 to less than about 1.9 wt% based on the total weight of the vapour precursor material. In one embodiment nicotine is present in an amount of from about 1 to less than about 1.8 wt% based on the total weight of the vapour precursor material.

In one embodiment, the vapour precursor material(s) may contain one or acids. In some embodiments, the vapour precursor material(s) may contain one or more acids in addition to nicotine (as the active agent). In some embodiments, the one or more acids may be one or more organic acids. In some embodiments, the one or more acids may be one or more organic acids selected from the group consisting of benzoic acid, levulinic acid, malic acid, maleic acid, fumaric acid, citric acid, lactic acid, acetic acid, succinic acid, and mixtures thereof. When included in the vapour precursor material(s) in combination with nicotine, the one or more acids may provide a formulation in which the nicotine is at least partially in protonated (such as monoprotonated and/or diprotonated) form.

In order to address various issues and advance the art, this disclosure shows by way of illustration, various embodiments in which the claimed invention may be practiced. The advantages and features of the disclosure are of a representative sample of embodiments only, and are not exhaustive and/or exclusive. They are presented only to assist in understanding and to teach the claimed invention.

It is to be understood that advantages, embodiments, examples, functions, features, structures and/or other aspects of the disclosure as defined by the claims or limitations on equivalents to the claims, and that other embodiments may be utilised and modifications may be made without departing from the scope of the claims. Various embodiments may suitable comprise, consist of, or consist essentially of, various combinations of the disclosed elements, components, features, parts, steps, means, etc. other than those specifically described herein.

## Claims

1. A cartomiser for a vapour provision system configured to selectively generate vapours with different flavour characteristics, wherein the cartomiser comprises:
(i) a reservoir comprising a first vapour precursor material (3) having a first flavour
characteristic, and a second vapour precursor material (2) having a second
flavour characteristic; and
(ii) at least one vaporiser (4,5) for generating vapour from the first vapour precursor material and the second vapour precursor material;
wherein the first vapour precursor material and the second vapour precursor material are configured to form an inhomogeneous mixture within the reservoir; wherein the first vapour precursor material is configured to provide vapour with the first flavour characteristic; and wherein the second vapour precursor material is configured to provide vapour with the second flavour characteristic, wherein the second flavour characteristic is different from the first flavour characteristic.

2. The cartomiser of claim 1, wherein the second vapour precursor material is configured to provide vapour after at least a portion of the first vapour precursor material has been vaporized.

3. The cartomiser of claim 1 or claim 2, wherein the second vapour precursor material is configured to provide vapour after at least 90% of the first vapour precursor material has been vaporized.

4. The cartomiser of any one of claims 1 to 3, wherein the cartomiser comprises a single vaporiser for generating vapour from both the first vapour precursor material and the second vapour precursor material.

5. The cartomiser of any one of claims 1 to 4, wherein the first and second vapour precursor materials are immiscible, preferably wherein the first vapour precursor material is aqueous and the second vapour precursor material comprises an oil.

6. The cartomiser of any one of claims 1 to 4, wherein the first vapour precursor material is a different state of matter to the second vapour precursor material.

7. The cartomiser of claim 6, wherein the second vapour precursor material is a solid, preferably a solid having a melting temperature below about 200°C.

8. The cartomiser of claim 7, wherein the second vapour precursor material forms a coating on a substrate or is impregnated into a substrate.

9. The cartomiser of any one of claims 4 to 8, wherein the second vapour precursor material is less dense than the first vapour precursor material.

10. The cartomiser of any one of claims 1 to 9, wherein the first vapour precursor material comprises at least one of propylene glycol and glycerol, and the second flavour characteristic of the second vapour precursor material comprises a tobacco or non-tobacco flavour.

11. The cartomiser of any one of claims 1 to 10, wherein the reservoir further comprises a layer or barrier material which separates the first vapour precursor material from the second vapour precursor material, preferably wherein the second vapour precursor material is encapsulated by the barrier material.

12. The cartomiser of any one of claims 1 to 11, wherein the second vapour precursor material amounts to about 5 to about 10 wt% of the total material in the reservoir.

13. A vapour provision system comprising the cartomiser of any one of claim 1 to 12, preferably further comprising at least one tobacco material, wherein the second flavour characteristic of the second vapour precursor material comprises a tobacco or non-tobacco flavour.

14. Use of a first vapour precursor material and a second vapour precursor material in a vapour provision system, to provide an end-of-life flavour; wherein the first and second vapour precursor materials form an inhomogeneous mixture; wherein the first vapour precursor material is configured to provide vapour with a first flavour characteristic; wherein the second vapour precursor material is configured to provide vapour with a second flavour characteristic that is different from the first flavour characteristic; wherein the second vapour precursor material is configured to provide vapour after at least a substantial portion of the first vapour precursor material has been vaporized, and wherein the second flavour characteristic of the second vapour precursor material comprises a tobacco or non-tobacco flavour.

15. A method of operating a vapour provision system configured to selectively generate vapours with different flavour characteristics for inhalation by a user, wherein the method comprises generating a vapour from a first vapour precursor material having a first flavour characteristic, and generating a vapour from a second vapour precursor material having a second flavour characteristic that is different from the first flavour characteristic, wherein the first vapour precursor material and the second vapour precursor material are inhomogeneous, and wherein the second vapour precursor material is configured to provide vapour after at least a portion of the first vapour precursor material has been vaporized, or wherein the second vapour precursor material is configured to provide vapour with the second flavour characteristic in response to a user action on the vapour provision system.

## Patentansprüche

1. Cartomizer für ein Dampfbereitstellungssystem, der so ausgebildet ist, dass er Dämpfe mit verschiedenen Aromamerkmalen selektiv erzeugt, wobei der Cartomizer Folgendes umfasst:
(i) einen Tank, der ein erstes Dampfvorläufermaterial (3) mit einem ersten Aromamerkmal und ein zweites Dampfvorläufermaterial (2) mit einem zweiten Aromamerkmal umfasst; und
(ii) mindestens einen Verdampfer (4,5) zum Erzeugen von Dampf aus dem ersten Dampfvorläufermaterial und dem zweiten Dampfvorläufermaterial;
wobei das erste Dampfvorläufermaterial und das zweite Dampfvorläufermaterial so ausgebildet sind, dass sie innerhalb des Tanks eine inhomogene Mischung bilden; wobei das erste Dampfvorläufermaterial so ausgebildet ist, dass es Dampf mit dem ersten Aromamerkmal erzeugt; und wobei das zweite Dampfvorläufermaterial so ausgebildet ist, dass es Dampf mit dem zweiten Aromamerkmal erzeugt, wobei das zweite Aromamerkmal vom ersten Aromamerkmal verschieden ist.

2. Cartomizer nach Anspruch 1, wobei das zweite Dampfvorläufermaterial so ausgebildet ist, dass es Dampf bereitstellt, nachdem zumindest ein Teil des ersten Dampfvorläufermaterials verdampft worden ist.

3. Cartomizer nach Anspruch 1 oder Anspruch 2, wobei das zweite Dampfvorläufermaterial so ausgebildet ist, dass es Dampf bereitstellt, nachdem mindestens 90 % des ersten Dampfvorläufermaterials verdampft worden sind.

4. Cartomizer nach einem der Ansprüche 1 bis 3, wobei der Cartomizer einen einzelnen Verdampfer zum Erzeugen von Dampf sowohl aus dem ersten Dampfvorläufermaterial als auch aus dem zweiten Dampfvorläufermaterial umfasst.

5. Cartomizer nach einem der Ansprüche 1 bis 4, wobei das erste und das zweite Dampfvorläufermaterial nicht mischbar sind, wobei vorzugsweise das erste Dampfvorläufermaterial wässrig ist und das zweite Dampfvorläufermaterial ein Öl umfasst.

6. Cartomizer nach einem der Ansprüche 1 bis 4, wobei sich das erste Dampfvorläufermaterial in einem Aggregatzustand befindet, der von demjenigen des zweiten Dampfvorläufermaterials verschieden ist.

7. Cartomizer nach Anspruch 6, wobei das zweite Dampfvorläufermaterial ein Feststoff, vorzugsweise ein Feststoff mit einer Schmelztemperatur unterhalb von etwa 200 °C ist.

8. Cartomizer nach Anspruch 7, wobei das zweite Dampfvorläufermaterial eine Beschichtung auf einem Substrat bildet oder ein Substrat damit getränkt ist.

9. Cartomizer nach einem der Ansprüche 4 bis 8, wobei das zweite Dampfvorläufermaterial weniger dicht als das erste Dampfvorläufermaterial ist.

10. Cartomizer nach einem der Ansprüche 1 bis 9, wobei das erste Dampfvorläufermaterial Propylenglycol und/oder Glycerin umfasst und das zweite Aromamerkmal des zweiten Dampfvorläufermaterials ein Tabak- oder Nicht-Tabak-Aroma umfasst.

11. Cartomizer nach einem der Ansprüche 1 bis 10, wobei der Tank weiterhin ein Schicht- oder ein Sperrschichtmaterial umfasst, welches das erste Dampfvorläufermaterial vom zweiten Dampfvorläufermaterial trennt, wobei das zweite Dampfvorläufermaterial vorzugsweise vom Sperrschichtmaterial eingeschlossen wird.

12. Cartomizer nach einem der Ansprüche 1 bis 11, wobei das zweite Dampfvorläufermaterial etwa 5 bis etwa 10 Gew.-% des gesamten Materials im Tank beträgt.

13. Dampfbereitstellungssystem, das den Cartomizer nach einem der Ansprüche 1 bis 12 umfasst, das vorzugsweise weiterhin mindestens ein Tabakmaterial umfasst, wobei das zweite Aromamerkmal des zweiten Dampfvorläufermaterials ein Tabak- oder Nicht-Tabak-Aroma umfasst.

14. Verwendung eines ersten Dampfvorläufermaterials und eines zweiten Dampfvorläufermaterials in einem Dampfbereitstellungssystem, um ein Aroma am Ende der Lebensdauer bereitzustellen; wobei das erste und das zweite Dampfvorläufermaterial eine inhomogene Mischung bilden; wobei das erste Dampfvorläufermaterial so ausgebildet ist, dass es Dampf mit einem ersten Aromamerkmal bereitstellt; wobei das zweite Dampfvorläufermaterial so ausgebildet ist, dass es Dampf mit einem zweiten Aromamerkmal bereitstellt, das vom ersten Aromamerkmal verschieden ist; wobei das zweite Dampfvorläufermaterial so ausgebildet ist, dass es Dampf bereitstellt, nachdem zumindest ein wesentlicher Teil des ersten Dampfvorläufermaterials verdampft worden ist, und wobei das zweite Aromamerkmal des zweiten Dampfvorläufermaterials ein Tabak- oder Nicht-Tabak-Aroma umfasst.

15. Verfahren zum Betreiben eines Dampfbereitstellungssystems, das so ausgebildet ist, dass es Dämpfe mit verschiedenen Aromamerkmalen zur Inhalation durch einen Benutzer selektiv erzeugt, wobei das Verfahren das Erzeugen eines Dampfes aus dem ersten Dampfvorläufermaterial mit einem ersten Aromamerkmal und das Erzeugen eines Dampfes aus einem zweiten Dampfvorläufermaterial mit einem zweiten Aromamerkmal umfasst, das vom ersten Aromamerkmal verschieden ist, wobei das erste Dampfvorläufermaterial und das zweite Dampfvorläufermaterial inhomogen sind und wobei das zweite Dampfvorläufermaterial so ausgebildet ist, dass es Dampf bereitstellt, nachdem zumindest ein Teil des ersten Dampfvorläufermaterials verdampft worden ist, oder wobei das zweite Dampfvorläufermaterial so ausgebildet ist, dass es Dampf mit dem zweiten Aromamerkmal als Reaktion auf eine Einwirkung des Benutzers auf das Dampfbereitstellungssystem bereitstellt.

## Revendications

1. Cartomiseur pour un système de délivrance de vapeur configuré pour générer sélectivement des vapeurs avec différentes caractéristiques aromatiques, le cartomiseur comprenant :
(i) un réservoir comprenant un premier matériau précurseur de vapeur (3) ayant une première caractéristique aromatique, et un deuxième matériau précurseur de vapeur (2) ayant une deuxième caractéristique aromatique ; et
(ii) au moins un vaporisateur (4, 5) destiné à générer de la vapeur à partir du premier matériau précurseur de vapeur et du deuxième matériau précurseur de vapeur ;
dans lequel le premier matériau précurseur de vapeur et le deuxième matériau précurseur de vapeur sont configurés pour former un mélange inhomogène à l'intérieur du réservoir ; dans lequel le premier matériau précurseur de vapeur est configuré pour délivrer de la vapeur avec la première caractéristique aromatique ; et dans lequel le deuxième matériau précurseur de vapeur est configuré pour délivrer de la vapeur avec la deuxième caractéristique aromatique, la deuxième caractéristique aromatique étant différente de la première caractéristique aromatique.

2. Cartomiseur de la revendication 1, dans lequel le deuxième matériau précurseur de vapeur est configuré pour délivrer de la vapeur après qu'au moins une partie du premier matériau précurseur de vapeur a été vaporisée.

3. Cartomiseur de la revendication 1 ou la revendication 2, dans lequel le deuxième matériau précurseur de vapeur est configuré pour délivrer de la vapeur après qu'au moins 90 % du premier matériau précurseur de vapeur a été vaporisé.

4. Cartomiseur de l'une quelconque des revendications 1 à 3, le cartomiseur comprenant un seul vaporisateur destiné à générer de la vapeur à partir à la fois du premier matériau précurseur de vapeur et du deuxième matériau précurseur de vapeur.

5. Cartomiseur de l'une quelconque des revendications 1 à 4, dans lequel les premier et deuxième matériaux précurseurs de vapeur sont immiscibles, de préférence dans lequel le premier matériau précurseur de vapeur est aqueux et le deuxième matériau précurseur de vapeur comprend une huile.

6. Cartomiseur de l'une quelconque des revendications 1 à 4, dans lequel le premier matériau précurseur de vapeur est un état de la matière différent de celui du deuxième matériau précurseur de vapeur.

7. Cartomiseur de la revendication 6, dans lequel le deuxième matériau précurseur de vapeur est un solide, de préférence un solide ayant une température de fusion inférieure à environ 200 °C.

8. Cartomiseur de la revendication 7, dans lequel le deuxième matériau précurseur de vapeur forme un revêtement sur un substrat ou est imprégné à l'intérieur d'un substrat.

9. Cartomiseur de l'une quelconque des revendications 4 à 8, dans lequel le deuxième matériau précurseur de vapeur est moins dense que le premier matériau précurseur de vapeur.

10. Cartomiseur de l'une quelconque des revendications 1 à 9, dans lequel le premier matériau précurseur de vapeur comprend du propylène glycol et/ou du glycérol, et la deuxième caractéristique aromatique du deuxième matériau précurseur de vapeur comprend un arôme de tabac ou différent du tabac.

11. Cartomiseur de l'une quelconque des revendications 1 à 10, dans lequel le réservoir comprend en outre une couche ou un matériau barrière qui sépare le premier matériau précurseur de vapeur du deuxième matériau précurseur de vapeur, de préférence dans lequel le deuxième matériau précurseur de vapeur est encapsulé par le matériau barrière.

12. Cartomiseur de l'une quelconque des revendications 1 à 11, dans lequel le deuxième matériau précurseur de vapeur représente environ 5 à environ 10 % en poids de la totalité des matériaux dans le réservoir.

13. Système de délivrance de vapeur comprenant le cartomiseur de l'une quelconque des revendications 1 à 12, de préférence comprenant en outre au moins un matériau à base de tabac, dans lequel la deuxième caractéristique aromatique du deuxième matériau précurseur de vapeur comprend un arôme de tabac ou différent du tabac.

14. Utilisation d'un premier matériau précurseur de vapeur et d'un deuxième matériau précurseur de vapeur dans un système de délivrance de vapeur pour délivrer un arôme de fin de vie ; dans laquelle les premier et deuxième matériaux précurseurs de vapeur forment un mélange inhomogène ; dans laquelle le premier matériau précurseur de vapeur est configuré pour délivrer de la vapeur avec une première caractéristique aromatique ; dans laquelle le deuxième matériau précurseur de vapeur est configuré pour délivrer de la vapeur avec une deuxième caractéristique aromatique qui est différente de la première caractéristique aromatique ; dans laquelle le deuxième matériau précurseur de vapeur est configuré pour délivrer de la vapeur après qu'au moins une partie substantielle du premier matériau précurseur de vapeur a été vaporisée ; et dans laquelle la deuxième caractéristique aromatique du deuxième matériau précurseur de vapeur comprend un arôme de tabac ou différent du tabac.

15. Procédé de fonctionnement d'un système de délivrance de vapeur configuré pour générer sélectivement des vapeurs avec différentes caractéristiques aromatiques pour une inhalation par un utilisateur, le procédé comprenant la génération d'une vapeur à partir d'un premier matériau précurseur de vapeur ayant une première caractéristique aromatique, et la génération d'une vapeur à partir d'un deuxième matériau précurseur de vapeur ayant une deuxième caractéristique aromatique qui est différente de la première caractéristique aromatique, dans lequel le premier matériau précurseur de vapeur et le deuxième matériau précurseur de vapeur sont inhomogènes, et dans lequel le deuxième matériau précurseur de vapeur est configuré pour délivrer de la vapeur après qu'au moins une partie du premier matériau précurseur de vapeur a été vaporisée, ou dans lequel le deuxième matériau précurseur de vapeur est configuré pour délivrer de la vapeur avec la deuxième caractéristique aromatique en réponse à une action de l'utilisateur sur le système de délivrance de vapeur.
